(19) Europäisches Patentamt
European Patent Office
Office européen des brevets

(11) Veröffentlichungsnummer: 0 316 279
A2

# (12) EUROPÄISCHE PATENTANMELDUNG

(21) Anmeldenummer: 88810756.2

(22) Anmeldetag: 04.11.88

(51) Int. Cl.4: C 07 D 213/69
A 61 K 31/44

(30) Priorität: 10.11.87 CH 4376/87

(43) Veröffentlichungstag der Anmeldung:
17.05.89 Patentblatt 89/20

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

(72) Erfinder: Moerker, Theophile
Ergolzstrasse 72
CH-4414 Füllinsdorf (CH)

Peter, Heinrich, Dr.
Bündtenweg 69
CH-4102 Binningen (CH)

Patentansprüche für folgende Vertragsstaaten: ES + GR.

(54) **Veresterte azacyclische Hydroxyverbindungen.**

(57) 3-substituierte 4(1H)-Pyridinon-Verbindungen der Formel

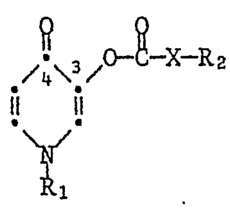

(I),

worin $R_1$ für einen gegebenenfalls substituierten Kohlenwasserstoffrest steht, X für eine Bindung oder eine Gruppe der Formel -O- oder -N($R_3$)-steht, worin $R_3$ Wasserstoff oder einen gegebenenfalls substituierten Kohlenwasserstoffrest bedeutet, und $R_2$ einen gegebenenfalls substituierten Kohlenwasserstoffrest darstellt, oder worin $R_2$ und $R_3$ zusammen einen gegebenenfalls substituierten, zweiwertigen Kohlenwasserstoffrest darstellen, und worin die restlichen Ringkohlenstoffatome des 4(1H)-Pyridinonrings, unabhängig voneinander, unsubstituiert oder durch einen gegebenenfalls substituierten Kohlenwasserstoffrest oder durch veräthertes oder verestertes Hydroxy substituiert sein können, und Salze von solchen Verbindungen bilden chelatartige Metallkomplexe, insbesondere mit trivalenten Metallionen und können u.a. als pharmakologische Wirkstoffe verwendet werden.

EP 0 316 279 A2

## Beschreibung

### Veresterte azacyclische Hydroxyverbindungen

Die Erfindung betrifft veresterte azacyclische Hydroxyverbindungen und Verfahren zu ihrer Herstellung, sowie ihre Verwendung und sie enthaltende pharmazeutische Zusammensetzungen.

Die Erfindung betrifft in erster Linie 3-substituierte 4(1H)-Pyridinon-Verbindungen der Formel

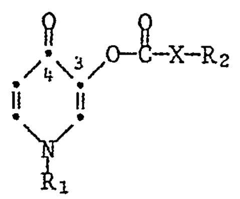 (I),

worin $R_1$ für einen gegebenenfalls substituierten Kohlenwasserstoffrest steht, X für eine Bindung oder eine Gruppe der Formel -O- oder -N($R_3$)-steht, worin $R_3$ Wasserstoff oder einen gegebenenfalls substituierten Kohlenwasserstoffrest bedeutet, und $R_2$ einen gegebenenfalls substituierten Kohlenwasserstoffrest darstellt, oder worin $R_2$ und $R_3$ zusammen einen gegebenenfalls substituierten, zweiwertigen Kohlenwasserstoffrest darstellen, und worin die restlichen Ringkohlenstoffatome des 4(1H)-Pyridinonrings, unabhängig voneinander, unsubstituiert oder durch einen gegebenenfalls substituierten Kohlenwasserstoffrest oder durch veräthertes oder verestertes Hydroxy substituiert sein können, und Salze von Verbindungen der Formel I mit salzbildenden Eigenschaften, ferner Verfahren zu ihrer Herstellung, ihre Verwendung, insbesondere als pharmakologisch wirksame Verbindungen, und pharmazeutische Präparate, die Verbindungen der Formel 1 oder Salze von solchen mit salzbildenden Eigenschaften enthalten.

Ein gegebenenfalls substituierter Kohlenwasserstoffrest $R_1$, $R_2$ und/oder $R_3$ kann einen cycloaliphatischen, cycloaliphatischaliphatischen, aromatischen oder araliphatischen, insbesondere jedoch einen aliphatischen Kohlenwasserstoffrest darstellen. Solche Reste sind in erster Linie Alkyl, wie Niederalkyl oder höheres Alkyl, ferner Alkenvl, wie Niederalkenyl, oder Alkinyl, wie Niederalkinyl, sowie Cycloalkyl, Cycloalkenyl, Cycloalkylalkyl, wie Cycloalkylniederalkyl, Phenyl, Naphthyl oder Phenylalkyl, wie Phenylniederalkyl.

Ein durch $R_2$ und $R_3$ zusammen gebildeter, gegebenenfalls substituierter, zweiwertiger Kohlenwasserstoffrest ist in erster Linie ein entsprechender aliphatischer Kohlenwasserstoffrest, wie Alkylen, z.B. Niederalkylen.

Substituenten von solchen Kohlenwasserstoffresten sind z.B. gegebenenfalls verethertes oder verestertes Hydroxy, wie Hydroxy, Alkoxy, z.B. Niederalkoxy, Alkanoyloxy, z.B. Niederalkanoyloxy, ferner ein Rest der Formel -O-C(=O)-X-$R_2$, worin X und $R_2$ die oben gegebenen Bedeutungen haben, sowie Halogen, gegebenenfalls substituiertes Amino, wie Amino, sowie Alkyl- oder Dialkylamino, z.B. Niederalkyl- oder Diniederalkylamino, ferner acyliertes Amino, wie der Rest der Formel -NH-C(=O)-X-$R_2$, worin X und $R_2$ die oben angegebenen Bedeutungen haben, oder gegebenenfalls verestertes oder amidiertes Carboxy, wie Carboxy, Alkoxycarbonyl, Z.B. Niederalkoxycarbonyl, oder gegebenenfalls substituiertes Aminocarbonyl, wie Aminocarbonyl oder N-Alkyl- oder N,N-Dialkyl-, z.B. N-Niederalkyl- oder N,N-Diniederalkylaminocarbonyl. Dabei sind in einem Rest $R_1$ bzw. $R_2$ nicht über ein Kohlenstoffatom gebundene Substituenten vorzugsweise durch mindestens zwei Kohlenstoffatome vom Ringstickstoffatom bzw. von X getrennt, sofern dieses von einer Bindung verschieden ist. Substituenten von cyclischen Resten, wie Cycloalkyl, Cycloalkenyl, Cycloalkylalkyl, Phenyl, Naphthyl oder Phenylalkyl, ferner von Niederalkylen, können ausser den obgenannten auch Alkyl-, wie Niederalkylreste sein. Substituierte Kohlenwasserstoffreste können einen oder mehrere, gleiche oder verschiedene Substituenten aufweisen.

Dabei bedeutet ein gegebenenfalls substituierter Kohlenwasserstoffrest $R_1$ in erster Linie ein gegebenenfalls substituierter aliphatischer, ferner araliphatischer Kohlenwasserstoffrest, insbesondere Alkyl, wie Nieder alkyl, ferner Phenylniederalkyl, wobei Substituenten eines solchen Restes insbesondere gegebenenfalls verethertes oder verestertes Hydroxy, wie Niederalkoxy, sowie Halogen, ferner gegebenenfalls substituiertes Amino, oder gegebenenfalls verestertes oder amidiertes Carboxy sind; dabei sind nicht über ein Kohlenstoffatom gebundene Substituenten vorzugsweise durch mindestens zwei Kohlenstoffatome vom Ringstickstoffatom getrennt sind.

Ein gegebenenfalls substituierter Kohlenwasserstoffrest $R_2$ ist in erster Linie ein entsprechender aliphatischer Kohlenwasserstoffrest, insbesondere Alkyl, wie Niederalkyl oder höheres Alkyl, wobei Substituenten vorzugsweise gegebenenfalls verestertes oder amidiertes Carboxy, insbesondere Alkoxycarbonyl, wie Niederalkoxycarbonyl, ferner gegebenenfalls verethertes oder verestertes Hydroxy oder gegebenenfalls substituiertes Amino bedeuten, wobei nicht über ein Kohlenstoffatom gebundene Substituenten vorzugsweise durch mindestens zwei Kohlenstoffatome von X getrennt sind, sofern dieses von einer Bindung verschieden ist.

Ein entsprechender Rest $R_3$ ist in erster Linie ein aliphatischer Kohlenwasserstoffrest, wie Alkyl, besonders Niederalkyl.

Ein durch $R_2$ und $R_3$ zusammen gebildeter zweiwertiger aliphatischer Kohlenwasserstoffrest, in erster Linie Alkylen, insbesondere Niederalkylen, kann als Substituenten u.a. vorzugsweise gegebenenfalls verestertes oder amidiertes Carboxy, insbesondere Alkoxycarbonyl, wie Niederalkoxycarbonyl enthalten.

Weitere, gegebenenfalls ein oder mehr als eines der übrigen Ringkohlenstoffatome des 4(1H)-Pyridinonrings substituierende, gegebenenfalls substituierte Kohlenwasserstoffreste sind in erster Linie entsprechende aliphatische Kohlenwasserstoffreste, wie Alkyl, besonders Niederalkyl, wobei Substituenten von solchen Resten insbesondere gegebenenfalls verethertes oder verestertes Hydroxy darstellen. Gegebenenfalls veretherte oder veresterte Hydroxygruppen als Substituenten von weiteren Ringkohlenstoffatome sind in erster Linie Niederalkoxy und Halogen.

Im Zusammenhang mit der vorliegenden Beschreibung haben Allgemeinbegriffe die folgenden bevorzugten Bedeutungen:
Unter den mit dem Ausdruck "Nieder" modifizierten Definitionen versteht man organische Reste und Verbindungen mit bis und mit acht, z.B. mit bis und mit drei, vorzugsweise vier Kohlenstoffatomen.

Alkyl ist z.B. Niederalkyl, wie Niederalkyl mit bis und mit drei Kohlenstoffatomen, z.B. n-Propyl oder Isopropyl, insbesondere Methyl, sowie Ethyl, n-Propyl oder Isopropyl, oder Niederalkyl mit vier bis und mit acht Kohlenstoffatomen, z.B. n-Butyl, Isobutyl, sek.-Butyl, tert.-Butyl, n-Pentyl, Neopentyl, n-Hexyl, n-Heptyl oder n-Octyl, oder auch höheres Alkyl, vorzugsweise mit 9 bis und mit 16 Kohlenstoffatomen, wie n-Nonyl, n-Decyl, n-Undecyl oder n-Dodecyl.

Alkenyl und Alkinyl sind vorzugsweise Niederalkenyl bzw. Niederalkinyl, jeweils mit bis und mit acht, vorzugsweise mit bis und mit vier Kohlenstoffatomen, wie Allyl, Methallyl oder Propargyl.

In Cycloalkyl-, Cycloalkenyl- und Cycloalkylalkylresten, worin Alkyl insbesondere für Niederalkyl steht, weist ein Cycloalkylrest vorzugsweise 3 bis 8, insbesondere 5 oder 6, und ein Cycloalkenyl insbesondere 5 oder 6 Ringkohlenstoffatome auf. Solche Reste sind z.B. Cyclopropyl, Cyclopentyl, Cyclohexyl, 1-Cyclohexenyl, Cyclopropylmethyl, Cyclopentylmethyl, Cyclohexylmethyl oder Cyclohexylethyl.

In einem Phenylalkylrest bedeutet Alkyl in erster Linie Niederalkyl; entsprechende Gruppen sind z.B. Benzyl oder 1- oder 2-Phenyläthyl.

Niederalkylen weist vorzugsweise 3 bis und mit 5 Kettenkohlenstoffatome auf und ist z.B. 1,3-Propylen, 1,4-Butylen oder 1,5-Pentylen.

Niederalkoxy ist z.B Methoxy, Ethoxy, n-Propyloxy, Isopropyloxy oder n-Butyloxy, während Niederalkanoyloxy z.B. für Acetoxy oder Propionyloxy, und Halogen (mit Halogenwasserstoffsäure verestertes Hydroxy) insbesondere für Chlor, ferner für Fluor oder Brom, sowie für Iod steht.

Niederalkyl- und Diniederalkylamino sind z.B. Methylamino, Ethylamino, Dimethylamino oder Diethylamino.

Niederalkoxycarbonyl ist z.B. Methoxycarbonyl, Ethoxycarbonyl, Propyloxycarbonyl oder Isopropyloxycarbonyl, während N-Niederalkyl- und N,N-Diniederalkyl-aminocarbonyl z.B. N-Methyl-aminocarbonyl, N-Ethyl-aminocarbonyl, N,N-Dimethyl-aminocarbonyl oder N,N-Diethyl-aminocarbonyl bedeuten.

Salze von Verbindungen der Formel I mit salzbildenden Eigenschaften sind in erster Linie pharmazeutisch verwendbare Salze, insbesondere mit salzbildenden Säuren, wie Mineralsäuren, z.B. Chlorwasserstoff- oder Schwefelsäure, oder starken organischen Carbon- oder Sulfonsäuren, z.B. Essig-, Fumar-, Embon- oder Methansulfonsäure, oder auch mit salzbildenden Basen, sofern solche Verbindungen z.B. freie Carboxygruppen als Substituenten aufweisen, wie Alkali- oder Erdalkali-, z.B. Natrium-, Kalium- oder Calciumsalze, oder Ammoniumsalze.

Je nach strukturellen Gegebenheiten können die Verbindungen der vorliegenden Erfindung in Form von Isomerengemischen oder von reinen Isomeren vorliegen.

Die neuen Verbindungen weisen wertvolle, insbesondere pharmakologisch verwertbare Eigenschaften auf. So können sie z.B. unter in vivo-Bedingungen in die entsprechenden 3-Hydroxy-4(1H)-pyridinon-Verbindungen umgewandelt werden, die stabile chelatartige Metallkomplexe, insbesondere mit trivalenten Metallionen, wie Chrom(III)-, Aluminium- und in erster Linie Eisen(III)-Ionen, bilden. Sie vermögen, wie z.B. im Tiermodell unter Verwendung der nicht-eisenüberladenen Gallenfistelratte in Dosen ab etwa 5 mg/kg gezeigt werden kann, u.a. die Ablagerung eisenhaltiger Pigmente im Gewebe zu verhindern und bei bestehenden Eisenablagerungen im Organismus eine Ausscheidung des Eisens bewirken, wie sie z.B. bei Hämochromatose, Hämosiderose, Lebercirrhose und Vergiftungen mit Verbindungen des dreiwertigen Eisens auftreten. Die neuen Verbindungen können deshalb als sog. Pro-Drugs für die entsprechenden 3-Hydroxy-4(1H)-pyridinon-Verbindungen bei der Behandlung von Krankheiten und krankhaften Zuständen des menschlichen und tierischen Körpers angewandt werden, die von einer übermässigen Belastung des Organismus mit Eisen begleitet sind, wie Thalassämie major, Sichelzell-Anämie, sidero achrestische Anämie, aplastische Anämie und weitere anämische Formen, bei denen Hämosiderose (d.h. eine lokale oder allgemeine Erhöhung der Eisenvorräte in sonst unbeschädigten Körpergeweben) eine Rolle spielt. Zu diesem Typus gehören auch krankhafte Zustände, die sich in Patienten nach mehrmaligen Bluttransfusionen oder mehrfach wiederholter Dialyse-Behandlung bei fehlender oder geschädigter Nierenfunktion entwickeln. Die neuen Verbindungen können auch bei Erkrankungen durch Eisen(III)-abhängige Mikroorganismen und Parasiten, wie insbesondere bei Malaria, eingesetzt werden. Ferner kann die Komplex-Bildung der entsprechenden 3-Hydroxy-4(1H)-pyridinone mit anderen dreiwertigen Metallen zu deren Ausscheidung aus dem Organismus verwendet werden, z.B. zur Entfernung von Aluminium bei der Dialyse-Encephalopathie und Osteomalacia, sowie bei der Alzheimer Krankheit. Verglichen mit den entsprechenden 3-Hydroxy-4(1H)-pyridinon-Verbindungen erweisen sich die neuen Verbindungen der vorliegenden Erfindung als oral wirksam und gut verträglich und zeigen eine langandauernde Wirkung.

Die Erfindung betrifft insbesondere Verbindungen der Formel I, worin X die oben gegebene Bedeutung hat, und $R_1$, $R_2$ und $R_3$, unabhängig voneinander, einen gegebenenfalls durch gegebenenfalls verethertes oder verestertes Hydroxy, gegebenenfalls substituiertes Amino und/oder gegebenenfalls verestertes oder amidiertes Carboxy substituierten

aliphatischen, cycloaliphatischen, cycloaliphatisch-aliphatischen, aromatischen oder araliphatischen Kohlenwasserstoffrest darstellen, wobei $R_2$ zudem für Wasserstoff stehen kann, oder worin $R_1$ die vorstehend gegebene Bedeutung hat, und $R_2$ und $R_3$ zusammen einen gegebenenfalls durch gegebenenfalls verethertes oder verestertes Hydroxy, gegebenenfalls substituiertes Amino und/oder gegebenenfalls verestertes oder amidiertes Carboxy substituierten zweiwertigen, aliphatischen Kohlenwasserstoffrest darstellen, und worin die übrigen Ringkohlenstoffatome des 4(1H)-Pyridinonrings, unabhängig voneinander, unsubstituiert sind oder gegebenenfalls verethertes oder verestertes Hydroxy, gegebenenfalls substituiertes Amino oder gegebenenfalls verestertes oder amidiertes Carboxy enthaltende aliphatische, cycloaliphatische, cycloaliphatischaliphatische, aromatische oder araliphatische Kohlenwasserstoffreste oder gegebenenfalls verethertes oder verestertes Hydroxy als Substituenten aufweisen können, und Salze, insbesondere pharmazeutisch verwendbare Salze von solchen Verbindungen mit salzbildenden Eigenschaften.

Die Erfindung betrifft in erster Linie Verbindungen der Formel I, worin X die oben gegebene Bedeutung hat, $R_1$ und $R_2$, unabhängig voneinander, Alkyl, z.B. Niederalkyl oder höheres Alkyl, Cycloalkyl, Cycloalkenyl, Cycloalkylniederalkyl, Phenyl, Naphthyl oder Phenylniederalkyl darstellen, wobei solche Reste gegebenenfalls durch Hydroxy, Niederalkoxy, Niederalkanoyloxy, den Rest der Formel -O-C(=O)-X-$R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiert sein können, $R_3$ Wasserstoff oder Niederalkyl bedeutet, oder worin $R_1$ die vorstehend gegebene Bedeutung hat, und $R_2$ und $R_3$ zusammen gegebenenfalls durch Hydroxy, Niederalkoxy, Niederalkanoyloxy, den Rest der Formel -O-C(=O)-X-$R_2$, worin X und $R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiertes Niederalkylen darstellen, und worin die übrigen Ringkohlenstoffatome des 4(1H)-Pyridinonrings, unabhängig voneinander, unsubstituiert sind oder, vorzugsweise die 2- oder die 6-Stellung, durch Alkyl, insbesondere Niederalkyl, substituiert sein können, wobei Alkyl, insbesondere Niederalkyl, durch Hydroxy, Niederalkoxy, verestertes Hydroxy der Formel -O-C(=O)-X-$R_2$, worin X und $R_2$ die vorstehend gegebenen Bedeutungen haben, und/oder durch Halogen substituiert sein kann, und Salze, insbesondere pharmazeutisch verwendbare Salze von solchen Verbindungen mit salzbildenden Eigenschaften.

Die Erfindung betrifft insbesondere Verbindungen der Formel I, worin X die oben gegebene Bedeutung hat, $R_1$ für gegebenenfalls in einer höheren als der 1-Stellung durch Niederalkoxy, vorzugsweise mit bis und mit vier Kohlenstoffatomen, z.B. Methoxy oder Ethoxy, substituiertes Niederalkyl, vorzugsweise mit bis und mit vier Kohlenstoffatomen, steht, $R_2$ für gegebenenfalls durch Niederalkoxycarbonyl, worin Niederalkoxy vorzugsweise bis und mit vier Kohlenstoffatome enthält, z.B. Methoxycarbonyl oder Ethoxycarbonyl, substituiertes Niederalkyl, vorzugsweise mit bis und mit acht Kohlenstoffatomen, z.B. Methyl, ferner Ethyl, n-Propyl oder n-Heptyl, steht, und $R_3$ Wasserstoff oder Niederalkyl, vorzugsweise mit bis und mit vier Kohlenstoffatomen, z.B. Methyl oder Ethyl, bedeutet, oder worin $R_1$ die oben gegebene Bedeutung hat, und $R_2$ und $R_3$ zusammen gegebenenfalls, vorzugsweise in 1-Stellung, durch Niederalkoxycarbonyl, worin Niederalkoxy vorzugsweise bis und mit vier Kohlenstoffatome enthält, z.B. Methoxycarbonyl oder Ethoxycarbonyl, substituiertes Niederalkylen mit 4 oder 5 Kohlenstoffatomen in der Kette bedeuten, und worin die übrigen Ringkohlenstoffatome des 4(1H)-Pyridinonrings, unabhängig voneinander, unsubstituiert sind oder, insbesondere die 6-oder bevorzugt die 2-Stellung, durch gegebenenfalls Hydroxy, Niederalkoxy, vorzugsweise mit bis und mit vier Kohlenstoffatomen, z.B. Methoxy oder Ethoxy, einen Rest der Formel -O-C(=O)-X-$R_2$, worin X und $R_2$ die vorstehend gegebenen Bedeutungen haben, oder Halogen, z.B. Chlor, enthaltendes Niederalkyl, vorzugsweise mit bis und mit vier Kohlenstoffatomen, besonders Methyl, ferner Ethyl, substituiert sein können, und Salze, insbesondere pharmazeutisch verwendbare Salze von solchen Verbindungen mit salzbildenden Eigenschaften.

Die Erfindung betrifft in erster Linie Verbindungen der Formel I, worin $R_1$ für Niederalkyl mit bis zu vier Kohlenstoffatomen, z.B. Methyl, Ethyl, n-Propyl, Isopropyl oder n-Butyl, steht, das in einer höheren als der 1-Stellung durch Niederalkoxy mit bis und mit vier Kohlenstoffatomen, z.B. Methoxy oder Ethoxy, substituiert sein kann (d.h. Niederalkoxy ist vom Ringstickstoffatom durch mindestens zwei Kohlenstoffatome getrennt), und worin X für eine direkte Bindung und $R_2$ für Niederalkyl mit vier bis und mit acht Kohlenstoffatomen, z.B. tert.-Butyl oder n-Heptyl, stehen, oder X eine Gruppe der Formel -O- und $R_2$ Niederalkyl mit bis und mit vier Kohlenstoffatomen bedeuten, oder vorzugsweise X eine Gruppe der Formel -N($R_3$)- darstellt, worin $R_3$ vorzugsweise Wasserstoff, ferner Niederalkyl mit bis und mit vier Kohlenstoffatomen, z.B. Methyl oder Ethyl, bedeutet, und $R_2$ gegebenenfalls durch Niederalkoxycarbonyl mit bis und mit fünf Kohlenstoffatomen, z.B. Methoxycarbonyl oder Ethoxycarbonyl, substituiertes Niederalkyl mit bis und mit vier Kohlenstoffatomen, vorzugsweise Methyl, bedeuten, oder worin $R_2$ und $R_3$ zusammen gegebenenfalls, vorzugsweise in 1-Stellung, durch Niederalkoxycarbonyl mit bis und mit fünf Kohlenstoffatomen, z.B. Methoxycarbonyl oder Ethoxycarbonyl, substituiertes Niederalkylen mit 4 oder 5 Kohlenstoffatomen in der Kette bedeuten, und worin die 2-Stellung durch Niederalkyl mit bis und mit vier Kohlenstoffatomen, z.B. Methyl, ferner Ethyl, substituiert ist, oder Salze, insbesondere pharmazeutisch verwendbare Salze von solchen Verbindungen mit salzbildenden Eigenschaften.

Die Erfindung betrifft insbesondere die in den Beispielen beschriebenen Einzelverbindungen.

Die Verbindungen der vorliegenden Erfindung können in an sich bekannter Weise hergestellt werden, in erster Linie indem man in einer Verbindung der Formel

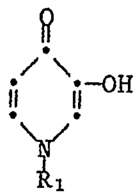

(II)

worin $R_1$ die oben gegebene Bedeutung hat, und die übrigen Ringkohlenstoffatome des 4(1H)-Pyridinonrings gegebenenfalls wie oben angegeben substituiert sein können, oder in einem reaktionsfähigen Derivat davon die Hydroxygruppe durch Behandeln mit einem, den Acylrest der Formel $-C(=O)-X-R_2$ einführenden Acylierungsmittel umsetzt, und, wenn erwünscht, ein verfahrensgemäss erhältliches Salz in die freie Verbindung überführt, und/oder eine verfahrensgemäss erhältliche freie, salzbildende Verbindung in ein Salz umwandelt, und/oder ein verfahrensgemäss erhältliches Gemisch von Isomeren auftrennt.

Reaktionsfähige Derivate von Verbindungen der Formel I sind insbesondere entsprechende Salze, wie Säureadditionssalze, z.B. mit geeigneten Mineralsäuren, wie Chlorwasserstoffsäure.

Die Veresterung der Hydroxygruppe kann in an sich bekannter Weise durchgeführt werden, vorzugsweise indem man das Ausgangsmaterial der Formel II mit einer, dem Acylrest der Formel $-(C=O)-X-R_2$ entsprechenden Säure oder mit einem geeigneten reaktionsfähigen Derivat davon umsetzt.

Reaktionsfähige Derivate der entsprechenden Säuren sind in erster Linie entsprechende Anhydride, wie symmetrische, gemischte und innere Anhydride von solchen Säuren. Unter den gemischten Anhydriden sind insbesondere die gemischten Anhydride mit starken anorganischen Säuren, vorzugsweise Halogenwasserstoffsäuren, wie Brom- und in erster Linie Chlorwasserstoffsäure (d.h. die entsprechenden Säurehalogenide, wie -bromide und in erster Linie -chloride), ferner mit Cyanwasserstoffsäure, sowie diejenigen mit Kohlensäurehalbestern, wie entsprechenden Kohlensäureniederalkylhalbestern, z.B. -methylhalbestern, -ethylhalbestern oder -isobutylhalbestern (die man z.B. durch Umsetzen eines geeigneten Salzes der Säure, z.B. eines Ammoniumsalzes davon, mit einem entsprechenden Chlorameisensäureester erhalten kann), oder mit geeignet substituierten Niederalkancarbonsäuren, insbesondere einer Halogen-substituierten Essigsäure, z.B. Trichloressigsäure (die man z.B. durch Behandeln der Säure oder eines geeigneten Salzes davon mit dem entsprechenden Säurehalogenid z.B. -chlorid, erhalten kann) zu verstehen. Innere Anhydride sind u.a. die entsprechenden Ketene oder, als innere Anhydride von Carbaminsäureverbindungen, die entsprechenden Isocyanatoverbindungen.

Weitere reaktionsfähige Säurederivate sind aktivierte Ester, wie Ester mit vinylogen Alkoholen (d.h. Enolen), z.B. Ester mit vinylogen Niederalkanolen, mit N-Hydroxy-imidverbindungen, wie N-Hydroxysuccinimid, mit eine Elektronen-anziehende Gruppe aufweisenden Niederalkanolen, z.B. Cyanmethanol, oder mit geeignet substituierten Phenolen, z.B. Pentachlorphenol oder 2,4-Dinitrophenol.

Die Acylierungsreaktion wird üblicherweise in Gegenwart von Kondensationsmitteln durchgeführt, wobei man bei Verwendung der freien Säure geeignete, Wasser-bindende Mittel, ferner Carbodiimide, z.B. Dicyclohexylcarbodiimid, bei Verwendung von Derivaten vorzugsweise Säurebindende Basen, besonders entsprechende organische Basen, z.B. Triethylamin oder Pyridin, einsetzt. Man arbeitet vorzugsweise in Gegenwart von geeigneten Lösungsmitteln oder Gemischen davon, wobei dafür auch Säurebindende Basen in Frage kommen können, wenn notwendig, unter Kühlen oder Erwärmen, in einem geschlossenen Gefäss und/oder in der Atmosphäre eines Inertgases, z.B. Stickstoff oder Argon.

Ausser der 3-Hydroxygruppe können auch andere, im Ausgangsmaterial vorhandene Hydroxy- und/oder Aminogruppen entsprechend acyliert werden. Dabei können verschiedenartig gebundene Hydroxygruppen stufenweise acyliert werden; so können z.B. aliphatisch gebundene Hydroxygruppen, im Vergleich zur 3-Hydroxygruppe, üblicherweise erst nach dieser und deshalb nur bei Einsatz eines Ueberschusse des Acylierungsmittels ebenfalls acyliert.

Die Ausgangsstoffe der Formel II sind bekannt oder können in an sich bekannter Weise hergestellt werden.

Verfahrensgemäss erhältliche Salze von Verbindungen der Formel 1 können in an sich bekannter Weise in die freien Verbindungen übergeführt werden, Säureadditionssalze durch Behandeln mit einem basischen Mittel, wie einer anorganischen Base, z.B. einem Alkali- oder Erdalkalimetall-, z.B. Natrium- oder Kaliumhydroxid, -carbonat oder -hydrogencarbonat, Salze mit Basen durch Behandeln mit einem sauren Reagens, wie einer Mineralsäure, z.B. Salz- oder Schwefelsäure.

Verfahrensgemäss erhältliche freie Verbindungen der Formel 1 können in an sich bekannter Weise in ihre Salze übergeführt werden, Verbindungen mit basischen Eigenschaften durch Behandeln mit Säuren oder geeigneten Derivaten davon, Verbindungen mit sauren Eigenschaften durch Behandeln mit einer geeigneten Base oder einem Derivat davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon verwendet wird.

Die vorliegende Erfindung betrifft ebenfalls pharmazeutische Präparate, die als Wirkstoff eine der pharmakologisch wirksamen Verbindungen der Formel I oder ein Salz davon enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartof felstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 %- bis 20 % vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulver förmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung von Krankheitszuständen, die, wie oben beschrieben, mit einem Ueberschuss z.B. von Eisen(III) oder Aluminium im Körper verbunden sind. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 10 kg eine tägliche Dosis von etwa 0,1 g bis etwa 10 g,

vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Celsiusgraden angegeben.

Beispiel 1:

Eine Suspension von 1,39 g 3-Hydroxy-1,2-dimethyl-4(1H)-pyridinon in 100 ml Acetonitril und 100 ml Methylenchlorid wird bei Raumtemperatur und unter Rühren mit 1,4 ml Triethylamin und dann mit 1,7 ml Isocyanatoessigsäureethylester versetzt, wobei sich das Reaktionsgemisch leicht erwärmt. Nach etwa 15 Minuten bildet sich eine Lösung, die nach weiteren 45 Minuten unter vermindertem Druck eingedampft wird. Der unter Hochvakuum getrocknete Rückstand kristallisiert aus einem Gemisch von Methylenchlorid und Essigsäureäthylester. Das Rohkristallisat wird in 10 ml Methanol aufgenommen, das unlösliche Material abfiltriert, das Filtrat unter vermindertem Druck eingedampft und der Rückstand aus einem Methylenchlorid/Essigsäureäthylester-Gemisch kristallisiert. Man erhält so das 3-(Ethoxycarbonylmethylaminocarbonyloxy)-1 2-dimethyl-4(1H)-pyridinon mit dem Schmelzpunkt von 132-135°.

Beispiel 2:

Eine Suspension von 5, 56 g 3-Hydroxy-1 2-dimethyl-4(1H)-pyridinon in 100 ml Acetonitril und 100 ml Methylenchlorid wird unter Rühren und bei Raumtemperatur mit 11,2 ml Triethylamin, dann mit 7,64 ml Chlorameisensäureethylester versetzt. Innerhalb von 15 Minuten bildet sich eine vollständige Lösung, und nach weiteren 45 Minuten werden die flüchtigen Anteile unter vermindertem Druck eingedampft. Der Rückstand wird unter Hochvakuum getrocknet, und das nicht umgesetzte Ausgangsmaterial aus Essigsäureethylester kristallisiert. Man erhält aus der Mutterlauge das 3-Ethoxycarbonyloxy-1,2-dimethyl-4(1H)-pyridinon in Form eines gelben Schaumes, Smp. 113-115°; Dünnschichtchromatogramm: Rf = 0,05 (7:3-Gemisch von Methylenchlorid und Aceton), 0,10 (9:1-Gemisch von Methylenchlorid und Isopropanol), 0,20 (9:1-Gemisch von Methylenchlorid und Methanol) und 0,35 (4:1-Gemisch von Methylenchlorid und Methanol).

Beispiel 3:

Eine Suspension von 0,556 g 3-Hydroxy-1,2-dimethyl-4(1H)-pyridinon in 40 ml Acetonitril und 40 ml Methylenchlorid wird unter Rühren und bei Raumtemperatur mit 0,56 ml Triethylamin, dann mit 1,02 ml Caprylsäurechlorid versetzt. Innerhalb von drei Minuten bildet sich eine Lösung; nach 30 Minuten ist die Reaktion beendet, und die flüchtigen Anteile werden unter vermindertem Druck verdampft. Der Rückstand wird unter Hochvakuum getrocknet und aus einem Gemisch von Methylenchlorid und Essigsäureethylester kristallisiert. Das so erhältliche 3-(n-Octanoyloxy)-1,2-dimethyl-4(1H)-pyridinon beginnt bei 140° zu sintern und zersetzt sich bei 180°.

Beispiel 4:

Eine Suspension von 0,556 g 3-Hydroxy-1,2-dimethyl-4(1H)-pyridinon in 40 ml Acetonitril und 40 ml Methylenchlorid wird unter Rühren und bei Raumtemperatur mit 0,56 ml Triethylamin, dann mit 0,76 ml Diethylcarbamoylchlorid versetzt. Nach 60 Minuten bleibt die Suspension bestehen; lt. Dünnschichtchromatogramm hat keine Reaktion stattgefunden. Das Gemisch wird mit 20 ml Pyridin versetzt, wobei es sich gelblich verfärbt. Nach weiteren 60 Minuten kann der Beginn der Reaktion festgestellt werden; diese wird durch Rühren während 16 Stunden zu Ende gebracht. Die flüchtigen Anteile werden unter vermindertem Druck verdampft, der Rückstand unter Hochvakuum getrocknet, dann in Methanol gelöst, die Lösung mit Aktivkohle versetzt, filtriert und eingedampft, und das Filtrat in 100 ml Essigsäureethylester aufgenommen. Dabei kristallisiert ein Produkt aus; eine weiteres Kristallisat kann durch Aufarbeiten der Mutterlauge erhalten werden. Die beiden Produkte werden vereinigt und in 50 ml Methylenchlorid gelöst. Die Lösung wird zweimal mit Wasser gewaschen und die organische Phase eingedampft. Der Rückstand wird aus Essigsäureethylester kristallisiert. Man erhält so das 3-(Diethylaminocarbonyloxy)-1,2-dimethyl-4(1H)-pyridinon, Smp. 135-137°.

Beispiel 5:

Eine Suspension des Hydrobromidsalzes von 3-Hydroxy-1,2-dimethyl-4(1H)-pyridinon in 100 ml Acetonitril und 100 ml Methylenchlorid wird unter Rühren und bei Raumtemperatur mit 8,4 ml Triethylamin, dann mit 80 ml einer Lösung von 3-Isocyanato-propionsäureethylester in Toluol (frisch aus β-Alaninethylester-hydrochlorid und Phosgen nach dem unten beschriebenen Verfahren hergestellt) versetzt. Innerhalb von 30 Minuten bildet sich eine Lösung; nach weiteren 30 Minuten ist die Reaktion beendet, und die flüchtigen Anteile werden unter vermindertem Druck verdampft. Der Rückstand wird unter Hochvakuum getrocknet und aus Essigsäureethylester kristallisiert. Man erhält so das Hydrobromid von 3-[(2-Ethoxycarbonyl-ethyl)-aminocarbonyloxy]-1,2-dimethyl-4(1H)-pyridinon, Smp. 153-154°, das in 100 ml Wasser gelöst wird. Der pH-Wert der Lösung wird durch Zugabe von Natriumhydrogencarbonat auf 7,5 gestellt und mit Methylenchlorid extrahiert. Der Rückstand aus dem organischen Extrakt wird aus Essisäureethylester umkristallisiert und ergibt das 3-[(2-Ethoxycarbonylethyl)-aminocarbonyloxy]-1 2-dimethyl-4( 1H)-pyridinon, Smp. 171-173°.

Die im obigen Verfahren verwendete Lösung von 3-Isocyanato-propionsäureethylester in Toluol kann wie folgt hergestellt werden: Ein Gemisch von 15,36 g ß-Alaninethylester in 600 ml Toluol wird unter Erwärmen auf 90° zur Lösung gebracht. Dann werden unter einer Argonatmosphäre und bei einer Temperatur von 90-95° innerhalb von 10 Minuten 63 ml einer 20%-igen Lösung von Phosgen in Toluol zugegeben. Durch Rühren bei etwa 90° während 16 Stunden erhält man die gewünschte Lösung des 3-Isocyanato-propionsäureethylesters in Toluol, die direkt in der obigen Reaktion eingesetzt wird.

Beispiel 6:

Zu einer Aufschlämmung von 0,945 g des Hydrochlorids von 1-Ethyl-3-hydroxy-2-methyl-4(1H)-pyridinon in 30 ml Acetonitril und 30 ml Methylenchlorid werden 1,40 ml Triethylamin zugegeben, und das Gemisch mit 1,135 ml Isocyanatoessigsäureethylester versetzt. Das Reaktionsgemisch erwärmt sich leicht, wobei innerhalb von 10 Minuten eine Lösung entsteht; die Reaktion ist nach 1,5 Stunden beendet. Das Reaktionsgemisch wird unter vermindertem Druck zur Trockne eingedampft, der Rückstand unter Hochvakuum getrocknet und aus Essigsäureethylester kristallisiert; man erhält so das Hydrochlorid von 1-Ethyl-3-(ethoxycarbonylmethylaminocarbonyloxy)-2-methyl-4(1H)-pyridinon, Smp. 154-156° (enthält noch Triethylamin). 2 g des Salzes werden in 20 ml Wasser gelöst, die Lösung mit Phosphorsäure auf pH 2,5 gestellt und mit Natriumchlorid gesättigt, dann mit Methylenchlorid extrahiert. Der Rückstand des organischen Extrakts wird aus einem 95:5-Gemisch von Essigsäureethylester und Methylenchlorid umkristallisiert und ergibt das 1-Ethyl-3-(ethoxycarbonylmethylaminocarbonyloxy)-2-methyl-4(1H)-pyridinon, Smp. 102-103°.

Das Ausgangsmaterial kann wie folgt erhalten werden: Eine Lösung von 21.62 g 3-Benzyloxy-2-methyl-4H-pyran-4-on in 500 ml Methanol wird mit 10,19 g Ethylamin-hydrochlorid in 200 ml Wasser, dann mit 8,0 g Natriumhydroxid in 100 ml Methanol versetzt. Die klare, gelbe Lösung wird während 5 Stunden unter Rückfluss erhitzt und gerührt, dann unter vermindertem Druck eingedampft. Der Rückstand wird in Methylenchlorid aufgenommen, die Lösung mit Wasser gewaschen und auf eine Silikagelkolonne aufgetragen. Mit einem 9:1-Gemisch vom Methylenchlorid und Methanol wird das amorphe 3-Benzyloxy-1-ethyl-2-methyl-4(1H)-pyridinon eluiert, in 100 ml konzentrierter wässriger Chlorwasserstoffsäure aufgenommen und während 3 Minuten unter Rückfluss gekocht, wobei eine Lösung entsteht. Diese wird unter Zusatz von Isopropanol eingedampft, und der Rückstand aus Isopropanol kristallisiert und mit Essigsäureethylester gewaschen. Das so erhältliche Hydrochlorid von 1-Ethyl-3-hydroxy-2-methyl-4(1H)-pyridinon schmilzt bei 196-198°.

Beispiel 7:

Eine Aufschlämmung von 2,036 g 3-Hydroxy-2-methyl-1-n-propyl-4(1H)-pyridinon-hydrochlorid in 50 ml Acetonitril und 50 ml Methylenchlorid wird bei Raumtemperatur mit 2,80 ml Triethylamin, dann mit 2,27 ml Isocyanatoessigsäureethylester versetzt. Die Reaktion verläuft leicht exotherm, es entsteht innerhalb von 10 Minuten eine Lösung, und die Reaktion ist nach 1,5 Stunden beendet. Die Lösung wird unter vermindertem Druck zur Trockne genommen, der Rückstand unter Hochvakuum getrocknet und aus Essigsäureethylester kristallisiert; man erhält so das Hydrochlorid von 3-(Ethoxycarbonylmethylaminocarbonyloxy)-2-methyl-1-n-propyl-4(1H)-pyridinon, Smp. 139-141°, das mit Triethylaminhydrochlorid verunreinigt ist.

Eine Lösung von 4,1 g des Salzes in 40 ml Wasser wird bis zu pH 2,5 mit Phosphorsäure behandelt, mit Natriumchlorid gesättigt und mit Methylenchlorid extrahiert. Der Rückstand aus dem organischen Extrakt wird aus einem 95:5-Gemisch von Essigsäureethylester und Methylenchlorid kristallisiert und ergibt das 3-(Ethoxycarbonylmethylaminocarbonyloxy)-2-methyl-1-n-propyl-4(1H)-pyridinon, Smp. 96-97°.

Das Ausgangsmaterial kann wie folgt hergestellt werden:

Eine Lösung von 43,24 g 3-Benzyloxy-2-methyl-4H-pyran-4-on in 1000 ml Methanol wird mit 20,64 ml n-Propylamin, dann mit einer Lösung von 12,0 g Natriumhydroxid in 100 ml Methanol versetzt. Das Reaktionsgemisch wird während drei Stunden unter Rückfluss gekocht, dann eingedampft, und der Rückstand in Methylenchlorid aufgenommen. Die organische Lösung wird mit Wasser gewaschen, die Phasen durch Zugabe von gesättigter Natriumchloridlösung in Wasser geklärt, und der organische Extrakt über Natriumsulfat getrocknet und unter vermindertem Druck eingedampft. Der Rückstand wird unter Hochvakuum getrocknet und ergibt das 3-Benzyloxy-2-methyl-1-n-propyl-4(1H)-pyridinon, das als öliges Produkt vorliegt.

Eine Lösung von 10 g 3-Benzyloxy-2-methyl-1-n-propyl-4(1H)-pyridinon in 100 ml konzentrierter (37 %) wässriger Chlorwasserstoffsäure wird während 5 Minuten unter Rückfluss gekocht, wobei sich zunächst ein Niederschlag bildet, der wieder in Lösung geht. Das Reaktionsprodukt wird mit Isopropanol versetzt und unter vermindertem Druck bis zur Bildung von kristallinem Material eingedampft. Dieses wird aus Essigsäureethylester umkristallisiert und ergibt das 3-Hydroxy-2-methyl-1-n-propyl-4(1H)-pyridinon-hydrochlorid, Smp. 205-207°.

Beispiel 8:

Eine Suspension von 1,89 g 2-Ethyl-3-hydroxy-1-methyl-4(1H)-pyridinon-hydrochlorid in 50 ml Acetonitril und 50 ml Methylenchlorid wird bei Raumtemperatur mit 2,8 ml Triethylamin, dann mit 2,27 ml Isocyanatoessigsäureethylester versetzt. Die Reaktion verläuft leicht exotherm, es bildet sich eine Lösung, und die Reaktion ist nach 1,5 Stunden beendet. Das Reaktionsgemisch wird unter vermindertem Druck zur Trockne genommen, der Rückstand wird unter Hochvakuum getrocknet und aus Essigsäureethylester kristallisiert. Eine Lösung von 4,0 g des so erhältlichen Hydrochlorids von 3-(Ethoxycarbonylmethylaminocarbonyloxy)-2-ethyl-1-methyl-4(1H)-pyridinon in 40 ml Wasser wird mit Phosphorsäure auf pH 2.5 gestellt, mit Natriumchlorid gesättigt und mit Methylenchlorid extrahiert. Der Rückstand des organischen Extrakts wird aus einem 95:5-Gemisch von Essigsäureethylester und Methylenchlorid kristallisiert, und man erhält so das 3-(Ethoxycarbonylmethylaminocarbonyloxy)-2-ethyl-1-methyl-4(1H)-pyridinon, Smp. 99-100°.

Das Ausgangsmaterial kann wie folgt hergestellt werden:

Ein Gemisch von 350 g 2-Ethyl-3-hydroxy-4H-pyran-4-on in 3000 ml Methanol wird mit 108 g Natriumhydroxid in 400 ml Wasser versetzt, und die farblose Lösung mit 336 ml Benzylchlorid behandelt.

Man kocht während 6 Stunden unter Rückfluss, rührt während 16 Stunden bei Raumtemperatur weiter und verdampft das Lösungsmittel. Der Rückstand wird in 1000 ml einer Phosphatpufferlösung (pH 8,5> aufgenommen und zweimal mit je 600 ml Methylenchlorid extrahiert. Der aus dem Extrakt erhältliche Rückstand kann mittels Chromatographie an Silikagel unter Verwendung von Methylenchlorid als Eluierungsmittel oder mittels Destillation gereinigt werden.

Eine Lösung von 23 g des so erhältlichen 3-Benzyloxy-2-ethyl-4H-pyran-4-ons in 500 ml Methanol wird mit 8,44 g Methylamin-hydrochlorid in 200 ml Wasser, dann mit 8,5 g Natriumhydroxid versetzt. Das Reaktionsgemisch wird während 3 Tagen bei Raumtemperatur gerührt, dann unter vermindertem Druck eingedampft, und der Rückstand in Methylenchlorid aufgenommen; der unlösliche Anteil wird verworfen. Die klare Lösung wird an Silikagel chromatographiert, wobei mit einem 97:3-Gemisch von Methylenchlorid und Methanol eluiert wird.

Das so erhältliche 3-Benzyloxy-2-ethyl-1-methyl-4(1H)-pyridinon wird in 100 ml konzentrierter wässriger Chlorwasserstoffsäure aufgenommen und während 5 Minuten am Rückfluss gekocht, wobei eine Lösung entsteht. Diese wird unter Zusatz von Isopropanol eingedampft, und das so erhältliche 2-ethyl-3-hydroxy-1-methyl-4(1H)-pyridinon-hydrochlorid aus Isopropanol kristallisiert und mit Essigsäureethylester gewaschen, Smp. 178-179°.

Beispiel 9:

Ein Gemisch von 5,49 g 3-Hydroxy-1-(2-methoxyethyl)-2-methyl-4(1H)-pyridinon in 150 ml Acetonitril und 150 ml Methylenchlorid wird mit 5,60 ml Triethylamin und bei Raumtemperatur mit 6,83 ml Isocyanatoessigsäureethylester versetzt. Die leicht exotherme Reaktion, bei der sich innerhalb von 10 Minuten eine Lösung bildet, ist nach 3 Stunden beendet. Die Lösungsmittel werden unter vermindertem Druck entfernt, und der Rückstand unter Hochvakuum getrocknet und aus Essigsäureethylester kristallisiert; die Mutterlauge wird verworfen. 1 g des hellbeigen Produkts wird zwischen Methylenchlorid und Wasser verteilt; der Hauptteil des Produkts befindet sich in der Wasserphase, die eingedampft wird. Der Rückstand wird in Methanol gelöst, die Lösung mit Aktivkohle behandelt und eingedampft. Der Rückstand, aus Essigsäureethylester kristallisiert, ergibt das weisse kristalline 3-(Ethoxycarbonylmethylaminocarbonyloxy)-1-(2-methoxyethyl)-2-methyl-4(1H)-pyridinon, Smp. 134-135°.

Das Ausgangsmaterial kann wie folgt erhalten werden:

Eine Lösung von 43,24 g 3-Benzyloxy-2-methyl-4H-pyran-4-on in 800 ml Methanol wird mit 21,9 ml 2-Methoxy-ethylamin (98%ig), dann mit einer Lösung von 12,0 g Natriumhydroxid in 100 ml Methanol behandelt. Die gelbe Lösung wird während 5 Stunden unter Rückfluss gekocht, nochmals mit 4,4 ml 2-Methoxy-ethylamin versetzt, und während einer weiteren Stunde unter Rückfluss erhitzt. Nach dem Abkühlen wird unter vermindertem Druck eingedampft, der Rückstand in Methylenchlorid aufgenommen, und die Lösung mit Wasser gewaschen und eingedampft.

Ein Gemisch von 18,0 g des so erhältlichen 3-Benzyloxy-1-(2-methoxyethyl)-2-methyl-4(1H)-pyridinons und 100 ml rauchender wässriger Chlorwasserstoffsäure wird während 2 Minuten am Rückfluss gekocht, abgekühlt, mit Isopropanol versetzt und unter vermindertem Druck eingedampft. Das in Form eines kristallinen Rückstandes erhältliche 3-Hydroxy-1-(2-methoxyethyl)-2-methyl-4(1H)-pyridinon-hydrochlorid wird durch Umkristallisieren aus Essigsäureethylester und Isopropanol weitergereinigt.

Eine Lösung von 5 g des obigen Salzes in 50 ml Wasser wird durch Zugabe von festem Natriumhydroxid auf pH 7,0 gestellt, und das freie 3-Hydroxy--1-(2-methoxy-ethyl)-2-methyl-4(IH)-pyridinon-hydrochlorid dreimal mit je 200 ml Methylenchlorid extrahiert. Der Rückstand schmilzt nach Kristallisieren aus Essigsäureethylester bei 178-179°.

Beispiel 10:

Eine Aufschlämmung von 2,477 g .1-(3-Ethoxypropyl)-3-hydroxy-2-methyl-4(1H)-pyridinon-hydrochlorid in 50 ml Acetonitril und 50 ml Methylenchlorid wird mit 2,80 ml Triethylamin und bei Raumtemperatur mit 2,27 ml Isocyanatoessigsäureethylester versetzt. Es bildet sich innerhalb von 10 Minuten eine Lösung; die Reaktion ist nach 1,5 Stunden beendet und die flüchtigen Anteile werden unter vermindertem Druck entfernt. Der Rückstand wird unter Hochvakuum getrocknet und aus Essigsäureethylester kristallisiert.

Eine Lösung von 4,0 g des so erhältlichen 3-(Ethoxycarbonylmethylaminocarbonyloxy) -1-( 3-ethoxy-propyl)-2-methyl-4( 1H)-pyridinon-hydrochlorids, das bei 92-93° schmilzt und durch etwas Triethylaminhydrochlorid verunreinigt ist, in 40 ml Wasser wird mittels Phosphorsäure auf pH 2,5 gestellt, mit Natriumchlorid gesättigt und mit Methylenchlorid extrahiert. Der Rückstand wird aus einem 95:5-Gemisch von Essigsäureethylester und Methylenchlorid kristallisiert und ergibt das 3-(Ethoxycarbonylmethylaminocarbonyloxy)-1-(3-ethoxy-propyl)-2-methyl-4(1H)-pyridinon, das bei 84-85° schmilzt.

Das Ausgangsmaterial kann wie folgt erhalten werden:

Eine Lösung von 21,62 g 3-Benzyloxy-2-methyl-4H-pyran-4-on in 500 ml Methanol wird mit 15,0 ml 3-Ethoxypropylamin, dann mit einer Lösung von 6,0 g Natriumhydroxid in 100 ml Methanol versetzt. Die klare, gelbe Lösung wird während 5 Stunden unter Erwärmen zum Rückfluss gerührt, dann unter vermindertem Druck eingedampft. Der Rückstand wird in Methylenchlorid aufgenommen, die Lösung mit Wasser gewaschen und an Silikagel chromatographiert, wobei mit einem 9:1-Gemisch von Methylenchlorid und Methanol eluiert wird.

Das so erhältliche amorphe 3-Benzyloxy-1-(3-ethoxy-propyl)-2-methyl-4(1H)-pyridinon wird in 100 ml konzentrierter wässriger Chlorwasserstoffsäure aufgenommen, während 3 Minuten am Rückfluss gekocht, und die so erhältliche Lösung mit Isopropanol verdünnt und zur Trockne eingedampft. Der

Rückstand ergibt nach Kristallisieren aus Isopropanol und Waschen mit Essigsäureethylester das 1-(3-Ethoxy-propyl)-3-hydroxy-2-methyl-4(1H)-pyridinon-hydrochlorid, Smp. 130-131°.

Beispiel 11:

Eine Aufschlämmung von 2,78 g 1,2-Dimethyl-3-hydroxy-1(4H)-pyridinon in 100 ml Pyridin und 100 ml Acetonitril wird mit 2,80 ml Triethylamin versetzt und tropfenweise mit einer heissen Lösung von 1-Chlorcarbonyl-L-prolin-methylester-hydrochlorid in Toluol behandelt (letztere erhält man durch Umsetzen einer Lösung von 0,99 g L-Prolinmethylester-hydrochlorid (getrocknet) in 150 ml Toluol mit 245 ml einer 20%-igen Lösung von Phosgen in Toluol nach 6-stündigem Rühren bei 110°). Das Reaktionsgemisch verfärbt sich rötlich; es bildet sich eine Lösung, wobei sofort wieder ein Niederschlag entsteht. Nach 20 Stunden Reaktionszeit wird das feste Material abfiltriert, und das Filtrat unter vermindertem Druck eingedampft. Der Rückstand, der das gewünschte Produkt in Form von dessen Hydrochlorid enthält, wird in 50 ml Wasser gelöst, die Lösung wird durch Zugabe von Phosphorsäure auf pH 2,5 gestellt und mit Methylenchlorid extrahiert; nachträglich wird die wässrige Phase mit Natriumchlorid gesättigt und nochmals mit Methylenchlorid extrahiert. Der Rückstand des 2. Extrakts wird aus 10 ml Isopropanol und 50 ml Essigsäureethylester bei 4° kristallisiert und ergibt das 1,2-Dimethyl-3-[(2(S)-methoxycarbonyl-pyrrolidino)-carbonyloxy]-4(1H)-pyridinon-hydrochlorid, Smp. 198-200°.

Beispiel 12:

Eine Aufschlämmung von 5,56 g 3-Hydroxy-1,2-dimethyl-4(1H)-pyridinon in 400 ml Methylenchlorid und 400 ml Acetonitril wird mit 5,6 ml Triethylamin und dann innerhalb von 30 Minuten mit 7,38 ml Trimethylessigsäurechlorid versetzt. Die Reaktion ist nach 15 Minuten beendet, die entstandene Lösung wird unter vermindertem Druck eingedampft, der Rückstand in Wasser aufgenommen und mit Methylenchlorid extrahiert. Der Rückstand des organischen Extrakts wird aus 500 ml Essigsäureethylester kristallisiert und ergibt das 1,2-Dimethyl-3-pivaloyloxy-4(1H)-pyridinon, Smp. 112-113° (sintert ab 103°).

In analoger Weise können folgende Verbindungen erhalten werden:

3-(n-Octanoyloxy)-1-(2-methoxy-ethyl)-2-methyl-4(1H)-pyridinon;

3-(Ethoxycarbonyloxy)-1-(2-methoxy-ethyl)-2-methyl-4( 1H)-pyridinon;

3-( Diethylaminocarbonyloxy)-1-(2-methoxy--ethyl)-2-methyl-4(1H)-pyridinon;

3-[(2-Ethoxycarbonyl-ethyl)-aminocarbonyloxy]-1-(2-methoxy-ethyl)-2-methyl-4(1H)-pyridinon;

3-(n-Octanoyloxy)-1-(2-ethoxy-ethyl)-2-methyl-4(1H)-pyridinon;

3-(Ethoxycarbonyloxy)-1-(2-ethoxy-ethyl)-2-methyl-4(1H)-pyridinon;

3-(Diethylaminocarbonyloxy)-1-(2-ethoxy-ethyl)-2-methyl-4(H) -pyridinon;

3-(Ethoxycarbonylmethylaminocarbonyloxy)-1-(2-ethoxy-ethyl)-2-methyl-4(1H)-pyridinon; und

3-[(2-Ethoxycarbonyl-ethyl)-aminocarbonyloxy]-1-(2-ethoxy-ethyl)-2-methyl-4(1H)-pyridinon.

Beispiel 13:

Kapseln enthaltend 0,25 g Wirkstoff, z.B. einer der Verbindungen der Beispiele 1-12, können wie folgt hergestellt werden

Zusammensetzung (für 1000 Kapseln):

| | |
|---|---|
| Wirkstoff | 250 g |
| Talk | 36 g |
| Weizenstärke | 24 g |
| Magnesiumstearat | 16 g |
| Laktose | 4 g |
| | 330 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

**Patentansprüche**

1. Verbindungen der Formel

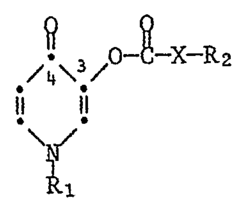

(I),

worin R1 für einen gegebenenfalls substituierten Kohlenwasserstoffrest steht, X für eine Bindung oder eine Gruppe der Formel -O- oder -($R_3$)-steht, worin $R_3$ Wasserstoff oder einen gegebenenfalls substituierten Kohlenwasserstoffrest bedeutet, und $R_2$ einen gegebenenfalls substituierten Kohlenwasserstoffrest darstellt, oder worin $R_2$ und $R_3$ zusammen einen gegebenenfalls substituierten, zweiwertigen Kohlenwasserstoffrest darstellen, und worin die restlichen Ringkohlenstoffatome des 4(1H)-Pyridinonrings, unabhängig voneinander, unsubstituiert oder durch einen gegebenenfalls substituierten Kohlenwasserstoffrest oder durch veräthertes oder verestertes Hydroxy substituiert sein können, und Salze von solchen Verbindungen mit salzbildenden Eigenschaften.

2. Verbindungen der Formel I gemäss Anspruch 1, worin $R_1$ und X die in Anspruch 1 gegebenen Bedeutungen haben, und worin $R_3$ Wasserstoff oder einen gegebenenfalls substi-

tuierten Kohlenwasserstoffrest bedeutet, und $R_2$ einen gegebenenfalls substituierten Kohlenwasserstoffrest darstellt, und Salze von solchen Verbindungen mit salzbildenden Eigenschaften.

3. Verbindungen der Formel 1 gemäss Anspruch 1, worin X die in Anspruch 1 gegebene Bedeutung hat, $R_1$ und $R_2$, unabhängig voneinander, Alkyl, Cycloalkyl, Cycloalkenyl, Cycloalkylniederalkyl, Phenyl, Naphthyl oder Phenylniederalkyl darstellen, wobei solche Reste gegebenenfalls durch Hydroxy, Niederalkoxy, Niederalkanoyloxy, den Rest der Formel -O-C(=O-X-$R_2$, worin X und $R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiert sein können, $R_3$ Wasserstoff oder Niederalkyl bedeutet, oder worin $R_1$ die vorstehend gegebene Bedeutung hat, und $R_2$ und $R_3$ zusammen gegebenenfalls durch Hydroxy, Niederalkoxy, Niederalkanoyloxy, den Rest der Formel -O-C(=O)-X-$R_2$, worin X und $R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiertes Niederalkylen darstellen, und worin die übrigen Ringkohlenstoffatome des 4(1H)-Pyridinonrings, unabhängig voneinander, unsubstituiert sind oder, vorzugsweise die 2- oder die 6-Stellung, durch Alkyl, insbesondere Niederalkyl, substituiert sein können, wobei Alkyl, insbesondere Niederalkyl, durch Hydroxy, Niederalkoxy, verestertes Hydroxy der Formel -0-C(=0)-X-$R_2$, worin X und $R_2$ die vorstehend gegebenen Bedeutungen haben, und/oder durch Halogen substituiert sein kann, und Salze von solchen Verbindungen salzbildenden Eigenschaften.

4. Verbindungen der Formel I gemäss Anspruch 1, worin X die im Anspruch 1 gegebene Bedeutung hat, $R_1$ und $R_2$, unabhängig voneinander, Alkyl, Cycloalkyl, Cycloalkenyl, Cycloalkylniederalkyl, Phenyl, Naphthyl oder Phenylniederalkyl darstellen, wobei solche Reste gegebenenfalls durch Hydroxy, Niederalkoxy, Niederalkanoyloxy, Halogen, Amino, Niederalkylamino, Diniederalkylamino, Carboxy, Niederalkoxycarbonyl, Carbamoyl, Niederalkylcarbamoyl oder Diniederalkylcarbamoyl substituiert sein können, $R_3$ Wasserstoff oder Niederalkyl bedeutet, und worin die übrigen Ringkohlenstoffatome des 4(1H)-Pyridinonrings, unabhängig voneinander, unsubstituiert sind oder durch Alkyl substituiert sein können, wobei Alkyl durch Hydroxy, Niederalkoxy, verestertes Hydroxy der Formel -O-C(=O)-X-$R_2$, worin X und $R_2$ die vorstehend gegebenen Bedeutungen haben, oder durch Halogen substituiert sein kann, und Salze von solchen Verbindungen mit salzbildenden Eigenschaften.

5. Verbindungen der Formel I gemäss Anspruch 1, worin $R_1$ für Niederalkyl mit bis zu vier Kohlenstoffatomen steht, das in einer höheren als der 1-Stellung durch Niederalkoxy mit bis und mit vier Kohlenstoffatomen substituiert sein kann, steht, und worin X für eine direkte Bindung und $R_2$ für Niederalkyl mit vier bis und mit acht Kohlenstoffatomen stehen, oder X eine Gruppe der Formel -O- und $R_2$ Niederalkyl mit bis und mit vier Kohlenstoffatomen bedeuten, oder X eine Gruppe der Formel -N($R_3$)- darstellt, worin $R_3$ Wasserstoff oder Niederalkyl mit bis und mit vier Kohlenstoffatomen bedeutet, und $R_2$ gegebenenfalls durch Niederalkoxycarbonyl mit bis und mit fünf Kohlenstoffatomen substituiertes Niederalkyl mit bis und mit vier Kohlenstoffatomen bedeuten, oder worin $R_2$ und $R_3$ zusammen gegebenenfalls durch Niederalkoxycarbonyl mit bis und mit fünf Kohlenstoffatomen substituiertes Niederalkylen mit 4 oder 5 Kohlenstoffatomen in der Kette bedeuten, und worin die 2-Stellung durch Niederalkyl mit bis und mit vier Kohlenstoffatomen substituiert ist, und Salze von solchen Verbindungen mit salzbildenden Eigenschaften.

6. Verbindungen der Formel I gemäss Anspruch 1, worin $R_1$ für Niederalkyl mit bis zu vier Kohlenstoffatomen steht, das in einer höheren als der 1-Stellung durch Niederalkoxy mit bis und mit vier Kohlenstoffatomen substituiert sein kann, und worin X für eine direkte Bindung und R9 für Niederalkyl mit vier bis und mit acht Kohlenstoffatomen stehen, oder X eine Gruppe der Formel -O- und $R_2$ Niederalkyl mit bis und mit vier Kohlenstoffatomen bedeuten, oder X eine Gruppe der Formel -N($R_3$)-, worin $R_3$ Wasserstoff oder Niederalkyl mit bis und mit vier Kohlenstoffatomen, und $R_2$ gegebenenfalls durch Niederalkoxycarbonyl mit bis und mit fünf Kohlenstoffatomen substituiertes Niederalkyl mit bis und mit vier Kohlenstoffatomen bedeuten, und worin die 2-Stellung durch Niederalkyl substituiert ist, und Salze von solchen Verbindungen mit salzbildenden Eigenschaften.

7. 3-(Ethoxycarbonylmethylaminocarbonyloxy)-1,2-dimethyl-4(1H)-pyridinon und Salze davon.

8. 3-(n-Octanoyloxy)-1,2-dimethyl-4(1H)-pyridinon und Salze davon.

9. 1-Ethyl-3-(ethoxycarbonylmethylaminocarbonyloxy)-2-methyl-4(1H)-pyridinon und Salze davon.

10. 3-(Ethoxycarbonylmethylaminocarbonyloxy)-1-(2-methoxyethyl)-2-methyl-4(1H)-pyridinon und Salze davon.

11. 3-Ethoxycarbonyloxy-1,2-dimethyl-4(1H)-pyridinon und 3-(Diethylaminocarbonyloxy)-1,2-dimethyl-4(1H)-pyridinon, sowie Salze davon.

12. 3-(Ethoxycarbonylmethylaminocarbonyloxy)-1-(3-ethoxy-propyl)-2-methyl-4(1H)-pyridinon, 3-(Ethoxycarbonylmethylaminocarbonyloxy-)-2-methyl-1-n-propyl-4(1H)-pyridinon, 3-(Ethoxycarbonylmethylaminocarbonyloxy)-2-ethyl-1-methyl-4(1H)-pyridinon, 1,2-Dimethyl-3-[(2(S)-methoxycarbonyl-pyrrolidino)-carbonyloxy]-4(1H)-pyridinon und 1,2-Dimethyl-3-pivaloyloxy-4(1H)-pyridinon, sowie Salze davon.

13. Pharmazeutisch verwendbare Salze von Verbindungen der Ansprüche 1, 3, 5, 9, 10 und 12 mit salzbildenden Eigenschaften.

14. Pharmazeutisch verwendbare Salze von Verbindungen der Ansprüche 2, 4, 6 bis 8 und 11 mit salzbildenden Eigenschaften.

15. Pharmazeutische Präparate enthaltend eine der Verbindungen der Ansprüche 1, 3, 5, 9, 10, 12 und 13.

16. Pharmazeutische Präparate enthaltend eine der Verbindungen der Ansprüche 2, 4, 6 bis 8, 11 und 14.

17. Verwendung der Verbindungen der Ansprüche 1, 3, 5, 9, 10, 12 und 13 zur Herstellung von pharmazeutischen Präparaten zur Behandlung des menschlichen oder tierischen Körpers.

18. Verwendung der Verbindungen der Ansprüche 2, 4, 6 bis 8, 11 und 14 zur Herstellung von pharmazeutischen Präparaten zur Behandlung des menschlichen oder tierischen Körpers.

19. Verfahren zur Herstellung von Verbindungen der Formel I gemäss Anspruch 1 oder Salzen von solchen Verbindungen mit salzbildenden Eigenschaften, dadurch gekennzeichnet, dass man in einer Verbindung der Formel

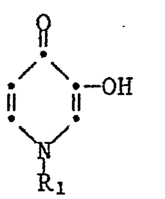

(II)

worin $R_1$ die in Anspruch 1 gegebene Bedeutung hat, und die übrigen Ringkohlenstoffatome des 4(1H)-Pyridinonrings gegebenenfalls wie im Anspruch 1 angegeben substituiert sein können, die Hydroxygruppe durch Behandeln mit einem, den Acylrest der Formel -C(=O)-X-$R_2$ einführenden Acylierungsmittel, worin X und $R_2$ die im Anspruch 1 gegebenen Bedeutungen haben, umsetzt, und, wenn erwünscht, ein verfahrensgemäss erhältliches Salz in die freie Verbindung überführt, und/oder eine verfahrensgemäss erhältliche freie, salzbildende Verbindung in ein Salz umwandelt, und/oder ein verfahrensgemäss erhältliches Gemisch von Isomeren auftrennt.

20. Verfahren zur Herstellung von Verbindungen gemäss Anspruch 2 oder Salzen von solchen Verbindungen mit salzbildenden Eigenschaften, dadurch gekennzeichnet, dass man in einer Verbindung der Formel II gemäss Anspruch 19, worin $R_1$ die im Anspruch 2 gegebene Bedeutung hat, und die übrigen Ringkohlenstoffatome des 4(1H)-Pyridinonrings gegebenenfalls wie im Anspruch 2 angegeben substituiert sein können, die Hydroxygruppe durch Behandeln mit einem, den Acylrest der Formel -C(=O)-X-$R_2$ einführenden Acylierungsmittel, worin X und $R_2$ die im Anspruch 2 gegebenen Bedeutungen haben, umsetzt, und, wenn erwünscht, ein verfahrensgemäss erhältliches Salz in die freie Verbindung überführt, und/oder eine verfahrensgemäss erhältliche freie, salzbildende Verbindung in ein Salz umwandelt, und/oder ein verfahrensgemäss erhältliches Gemisch von Isomeren auftrennt.

**Patentansprüche für folgende Vertragsstaaten:ES;GR**

1. Verfahren zur Herstellung von Verbindungen der Formel

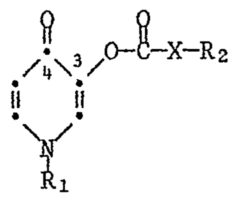

(I),

worin $R_1$ für einen gegebenenfalls substituierten Kohlenwasserstoffrest steht, X für eine Bindung oder eine Gruppe der Formel -O- oder -N($R_3$)-steht, worin $R_3$ Wasserstoff oder einen gegebenenfalls substituierten Kohlenwasserstoffrest bedeutet, und $R_2$ einen gegebenenfalls substituierten Kohlenwasserstoffrest darstellt, oder worin $R_2$ und $R_3$ zusammen einen gegebenenfalls substituierten, zweiwertigen Kohlenwasserstoffrest darstellen, und worin die restlichen Ringkohlenstoffatome des 4(1H)-Pyridinonrings, unabhängig voneinander, unsubstituiert oder durch einen gegebenenfalls substituierten Kohlenwasserstoffrest oder durch veräthertes oder verestertes Hydroxy substituiert sein können, oder Salzen von solchen Verbindungen mit salzbildenden Eigenschaften, dadurch gekennzeichnet, dass man in einer Verbindung der Formel

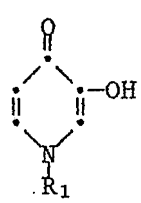

(II)

worin $R_1$ die in Anspruch 1 gegebene Bedeutung hat, und die übrigen Ringkohlenstoffatome des 4(1H)-Pyridinonrings gegebenenfalls wie im Anspruch 1 angegeben substituiert sein können, die Hydroxygruppe durch Behandeln mit einem, den Acylrest der Formel -C(=O)-X-$R_2$ einführenden Acylierungsmittel,

worin X und $R_2$ die im Anspruch 1 gegebenen Bedeutungen haben, umsetzt, und, wenn erwünscht, ein verfahrensgemäss erhältliches Salz in die freie Verbindung überführt, und/oder eine verfahrensgemäss erhältliche freie, salzbildende Verbindung in ein Salz umwandelt, und/oder ein verfahrensgemäss erhältliches Gemisch von Isomeren auftrennt.

2. Verfahren gemäss Anspruch 1, dadurch gekennzeichnet, dass man das Ausgangsmaterial der Formel II mit einer, dem Acylrest der Formel $-(C=O)-X-R_2$ entsprechenden Säure oder einem geeigneten reaktionsfähigen Derivat davon umsetzt.

3. Verfahren gemäss Anspruch 2, dadurch gekennzeichnet, dass man ein Anhydrid oder einen aktivierten Ester als Derivat einer, dem Acylrest der Formel $-(C=O)-X-R_2$ entsprechenden Säure verwendet.

4. Verfahren gemäss einem der Ansprüche 1 bis 3, dadurch gekennzeichnet, dass man Verbindungen der Formel I gemäss Anspruch 1, worin $R_1$ und X die in Anspruch 1 gegebenen Bedeutungen haben, und worin $R_3$ Wasserstoff oder einen gegebenenfalls substituierten Kohlenwasserstoffrest bedeutet, und $R_2$ einen gegebenenfalls substituierten Kohlenwasserstoffrest darstellt, oder Salze von solchen Verbindungen mit salzbildenden Eigenschaften herstellt.

5. Verfahren gemäss einem der Ansprüche 1-3, dadurch gekennzeichnet, dass man Verbindungen der Formel I gemäss Anspruch 1, worin X die in Anspruch 1 gegebene Bedeutung hat, $R_1$ und $R_2$, unabhängig voneinander, Alkyl, Cycloalkyl, Cycloalkenyl, Cycloalkylniederalkyl, Phenyl, Naphthyl oder Phenylniederalkyl darstellen, wobei solche Reste gegebenenfalls durch Hydroxy, Niederalkoxy, Niederalkanoyloxy, den Rest der Formel $-O-C(=O)-X-R_2$, worin X und $R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiert sein können, $R_3$ Wasserstoff oder Niederalkyl bedeutet, oder worin $R_1$ die vorstehend gegebene Bedeutung hat, und $R_2$ und $R_3$ zusammen gegebenenfalls durch Hydroxy, Niederalkoxy, Niederalkanoyloxy, den Rest der Formel $-O-C(=O)-X-R_2$, worin X und $R_2$ die vorstehenden Bedeutungen haben, ferner durch Halogen, Amino, Niederalkylamino, Diniederalkylamino, Carboxy, Niederalkoxycarbonyl, Carbamoyl, Niederalkylcarbamoyl und/oder Diniederalkylcarbamoyl substituiertes Niederalkylen darstellen, und worin die übrigen Ringkohlenstoffatome des 4(1H)-Pyridinonrings, unabhängig voneinander, unsubstituiert sind oder, vorzugsweise die 2- oder die 6-Stellung, durch Alkyl, insbesondere Niederalkyl, substituiert sein können, wobei Alkyl, insbesondere Nieder alkyl, durch Hydroxy, Niederalkoxy, verestertes Hydroxy der Formel $O-C(=O)-x-R_2$, worin X und $R_2$ die vorstehend gegebenen Bedeutungen haben, und/oder durch Halogen substituiert sein kann, oder Salze von solchen Verbindungen mit salzbildenden Eigenschaften herstellt.

6. Verfahren gemäss einem der Ansprüche 1-3, dadurch gekennzeichnet, dass man Verbindungen der Formel I gemäss Anspruch 1, worin X die im Anspruch 1 gegebene Bedeutung hat, $R_1$ und $R_2$, unabhängig voneinander, Alkyl, Cycloalkyl, Cycloalkenyl, Cycloalkylniederalkyl, Phenyl, Naphthyl oder Phenylniederalkyl darstellen, wobei solche Reste gegebenenfalls durch Hydroxy, Niederalkoxy, Niederalkanoyloxy, Halogen, Amino, Niederalkylamino, Diniederalkylamino, Carboxy, Niederalkoxycarbonyl, Carbamoyl, Niederalkylcarbamoyl oder Diniederalkylcarbamoyl substituiert sein können, $R_3$ Wasserstoff oder Niederalkyl bedeutet, und worin die übrigen Ringkohlenstoffatome des 4(1H)-Pyridinonrings, unabhängig voneinander, unsubstituiert sind oder durch Alkyl substituiert sein können, wobei Alkyl durch Hydroxy, Niederalkoxy, verestertes Hydroxy der Formel $-0-C(=0)-X-R_2$, worin X und $R_2$ die vorstehend gegebenen Bedeutungen haben, oder durch Halogen substituiert sein kann, oder Salze von solchen Verbindungen mit salzbildenden Eigenschaften herstellt.

7. Verfahren gemäss einem der Ansprüche 1-3, dadurch gekennzeichnet, dass man Verbindungen der Formel I gemäss Anspruch 1, worin $R_1$ für Niederalkyl mit bis zu vier Kohlenstoffatomen steht, das in einer höheren als der 1-Stellung durch Niederalkoxy mit bis und mit vier Kohlenstoffatomen substituiert sein kann, steht, und worin X für eine direkte Bindung und $R_2$ für Niederalkyl mit vier bis und mit acht Kohlenstoffatomen stehen, oder X eine Gruppe der Formel -O- und $R_2$ Niederalkyl mit bis und mit vier Kohlenstoffatomen bedeuten, oder X eine Gruppe der Formel $-N(R_3)-$ darstellt, worin $R_3$ Wasserstoff oder Niederalkyl mit bis und mit vier Kohlenstoffatomen bedeutet, und $R_2$ gegebenenfalls durch Niederalkoxycarbonyl mit bis und mit fünf Kohlenstoffatomen substituiertes Niederalkyl mit bis und mit vier Kohlenstoffatomen bedeuten, oder worin $R_2$ und $R_3$ zusammen gegebenenfalls durch Niederalkoxycarbonyl mit bis und mit fünf Kohlenstoffatomen substituiertes Niederalkylen mit 4 oder 5 Kohlenstoffatomen in der Kette bedeuten, und worin die 2-Stellung durch Niederalkyl mit bis und mit vier Kohlenstoffatomen substituiert ist, oder Salze von solchen Verbindungen mit salzbildenden Eigenschaften herstellt.

8. Verfahren gemäss einem der Ansprüche 1-3, dadurch gekennzeichnet, dass man Verbindungen der Formel I gemäss Anspruch 1, worin $R_1$ für Niederalkyl mit bis zu vier Kohlenstoffatomen steht, das in einer höheren als der 1-Stellung durch Niederalkoxy mit bis und mit vier Kohlenstoffatomen substituiert sein kann, und worin X für eine direkte Bindung $R_2$ für Niederalkyl mit vier bis und mit acht Kohlenstoffatomen stehen, oder X eine Gruppe der

Formel -O- und $R_2$ Niederalkyl mit bis und mit vier Kohlenstoffatomen bedeuten, oder X eine Gruppe der Formel -N($R_3$)-, worin $R_3$ Wasserstoff oder Niederalkyl mit bis und mit vier Kohlenstoffatomen, und $R_2$ gegebenenfalls durch Niederalkoxycarbonyl mit bis und mit fünf Kohlenstoffatomen substituiertes Niederalkyl mit bis und mit vier Kohlenstoffatomen bedeuten, und worin die 2-Stellung durch Niederalkyl substituiert ist, oder Salze von solchen Verbindungen mit salzbildenden Eigenschaften herstellt.

9. Verfahren gemäss einem der Ansprüche 1-3, dadurch gekennzeichnet, dass man 3-(Ethoxycarbonylmethylaminocarbonyloxy)-1,2-dimethyl-4(1H)-pyridinon oder Salze davon herstellt.

10. Verfahren gemäss einem der Ansprüche 1-3, dadurch gekennzeichnet, dass man 3-(n-Octanoyloxy)-1,2-dimethyl-4(1H)-pyridinon oder Salze davon herstellt.

11. Verfahren gemäss einem der Ansprüche 1-3, dadurch gekennzeichnet, dass man 1-Ethyl-3-(ethoxycarbonylmethylaminocarbonyloxy)-2-methyl-4(1H)-pyridinon oder Salze davon herstellt.

12. Verfahren gemäss einem der Ansprüche 1-3, dadurch gekennzeichnet, dass man 3-(Ethoxycarbonylmethylaminocarbonyloxy)-1-(2-methoxyethyl)-2-methyl-4(1H)-pyridinon oder Salze davon herstellt.

13. Verfahren gemäss einem der Ansprüche 1-3, dadurch gekennzeichnet, dass man 3-Ethoxycarbonyloxy-1,2-dimethyl-4(1H)-pyridinon oder 3-(Diethylaminocarbonyloxy)-1,2-dimethyl-4(IH)-pyridinon, sowie Salze davon herstellt.

14. Verfahren gemäss einem der Ansprüche 1-3, dadurch gekennzeichnet, dass man 3-(Ethoxycarbonylmethylaminocarbonyloxy)-2-methyl-1-n-propyl-4(1H)-pyridinon, 3-(Ethoxycarbonylmethylaminocarbonyloxy)-2-ethyl-1-methyl-4(1H)-pyridinon, 3-(Ethoxycarbonylmethylaminocarbonyloxy)-1-(3-ethoxy-propyl)-2-methyl-4(1H)-pyridinon, 1,2-Dimethyl-3-[(2(S)-methoxycarbonyl-pyrrolidino)-carbonyloxy]-4(1H)-pyridinon oder I,2-Dimethyl-3-pivaloyloxy-4(1H)-pyridinon, sowie Salze davon herstellt.